# FASCICULE DE BREVET EUROPEEN

(11) **EP 1 358 870 B1**
(45) Date de publication et mention de la délivrance du brevet: **14.06.2006**
(21) Numéro de dépôt: 03290847.7
(22) Date de dépôt: 04.04.2003
(51) Int. Cl.: A61K 8/04, A61K 8/37, A61K 8/898, A61Q 1/02

(54) **Fond de teint émulsion eau-dans-huile**
Wasser in Öl make-up Emulsion
Water in oil make-up emulsion

(30) Priorité: 02.05.2002 FR 0205512
(43) Date de publication de la demande: 05.11.2003
(73) Titulaire: L'OREAL, 75008 Paris (FR)
(72) Inventeur: Simonnet, Jean-Thierry, 75011 Paris (FR); Verloo, Aurore, 91570 Bievres (FR); Ozee, Emmanuelle, 94320 Thiais (FR)
(74) Mandataire: Kromer, Christophe

(56) Documents cités:
- EP-A- 1 086 687
- EP-A- 1 097 703
- FR-A- 2 776 183
- US-A- 5 942 213
- US-A- 5 976 510

## Description

La présente invention a pour objet une composition cosmétique de fond de teint fluide sous forme d'émulsion eau-dans huile comprenant un tensioactif siliconé et des pigments enrobés. L'invention a également pour objet un procédé de maquillage de la peau comprenant l'application du fond de teint sur la peau.

La composition de fond de teint est une composition de maquillage de la peau d'être humain. La composition selon l'invention peut être un fond de teint à appliquer sur le visage ou le cou, un produit anti-cernes, une crème teintée, un composition de maquillage du corps.

Les compositions de fond de teint sont couramment employées pour apporter une couleur esthétique à la peau, notamment au visage, mais également pour camoufler les imperfections de la peau telles que les rougeurs, les taches.

Il est connu du document FR-A-2686510 des émulsions fluides de fond de teint eau-dans-huile comprenant comme tensioactif ABIL® WE 09 vendu par la société Goldschmidt qui est un mélange de cétyl diméthicone copolyol, d'isostéarate de polyglycérol-4 et de laurate d'hexyle en proportion pondérale 40/30/30. Or on a constaté que les émulsions fluides préparées avec ce mélange ne sont pas stables au cours du temps : l'émulsion après un stockage de 4 mois à température ambiante (25 °C) peut relarguer de l'huile à la surface de la composition et n'est donc plus homogène. Sans agitation préalable, l'application d'une telle composition sur la peau laisse une sensation de gras nuisant à la qualité recherchée du fond de teint, notamment la facilité d'application sur la peau, le toucher agréable et la rapidité de séchage. La tenue de la matité du maquillage est altérée, le maquillage aparaissant brillant rapidement. Le maquillage ainsi obtenu n'est donc pas esthétique.

Le but de la présente invention est de disposer d'une composition de fond de teint ayant une bonne stabilité après un stockage à température ambiante (25 °C) pendant au moins 4 mois et permettant d'obtenir un maquillage homogène de la peau, présentant des qualités esthétiques satisfaisantes.

Les inventeurs ont découvert qu'un tel fond de teint pouvait être obtenu en utilisant un alkyl diméthicone copolyol et l'isostéarate de polyglycéryle-4 en des quantités particulière, et en utilisant également des pigments enrobés hydrophobes.

De façon plus précise, l'invention a pour objet un fond de teint sous forme d'émulsion eau-dans-huile comprenant une phase grasse, une phase aqueuse, un alkyl C₈-C₂₂ diméthicone copolyol, au moins 5 % en poids, par rapport au poids total de la composition, de pigments enrobés hydrophobes, la composition contenant de l'isostéarate de polyglycéryle-4 en une quantité telle que le rapport pondéral de l'alkyl C₈-C₂₂ diméthicone copolyol par rapport à l'isostéarate de polyglycéryle-4 est supérieur ou égal à 2.

L'invention a aussi pour objet un procédé cosmétique de maquillage non thérapeutique de la peau comprenant l'application sur la peau d'une composition telle que définie précédemment.

L'invention a également pour objet l'utilisation d'un alkyl C₈-C₂₂ diméthiconecopolyol dans une composition de fond de teint sous forme d'émulsion eau-dans-huile contenant au moins 5 % en poids, par rapport au poids total de la composition, de pigments enrobés hydrophobes, la composition contenant de l'isostéarate de polyglycéryle-4 en une quantité telle que le rapport pondéral de l'alkyl C₈-C₂₂ diméthiconecopolyol par rapport à l'isostéarate de polyglycéryle-4 est supérieur ou égal à 2, pour obtenir une émulsion stable et/ou homogène et/ou s'appliquant facilement sur la peau, et/ou pour obtenir un maquillage homogène de la peau.

L'émulsion selon l'invention présente une très bonne stabilité à température ambiante (25 °C), notamment après un stockage de 4 mois ou mieux de 6 mois, voire même de 8 mois. Le fond de teint s'applique facilement sur la peau, avec une sensation d'onctuosité, de douceur et non grasse, il sèche rapidement et se répartit de façon homogène sur la peau. Le maquillage obtenu présente une bonne tenue dans le temps de la matité.

L'alkyl C₈-C₂₂ diméthicone copolyol présent dans le fond de teint selon l'invention est un poly méthyl alkyl(C₈-C₂₂) diméthyl méthyl siloxane oxypropyléné et/ou oxyéthyléné.

L'alkyl C₈-C₂₂ diméthicone copolyol est avantageusement un composé de formule (I) suivante : 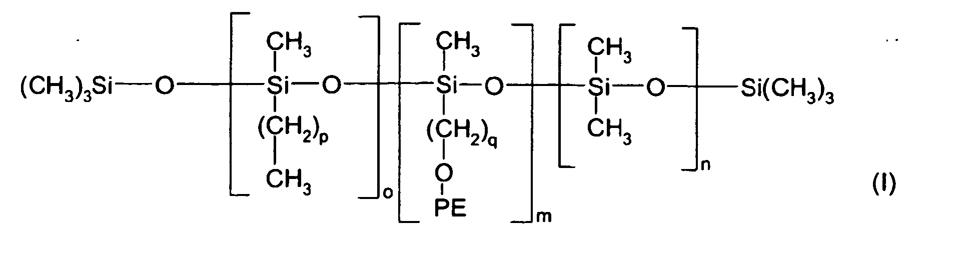 dans laquelle :
- PE représente (-C₂H₄O)ₓ-(C₃H₆O)_{y}-R, R étant choisi parmi un atome d'hydrogène et un radical alkyle de 1 à 4 atomes de carbone, x allant de 0 à 100 et y allant de 0 à 80, x et y n'étant pas simultanémént 0
- m va de 1 à 40
- n va de 10 à 200
- o va de 1 à 100
- p va de 7 à 21
- q va de 0 à 4
et de préférence :
R=H
m = 1 à 10
n = 10 à 100
o = 1 à 30
p = 15
q = 3

Comme alkyl C₈-C₂₂ diméthicone copolyol, on peut citer le cétyl diméthicone copolyol comme le produit commercialisé sous la dénomination Abil EM-90 par la société Goldschmidt.

L'alkyl C₈-C₂₂ diméthicone copolyol peut être présent dans l'émulsion selon l'invention en une teneur allant de 2 % à 10 % en poids, par rapport au poids total de l'émulsion, et de préférence allant de 2,5 % à 5 % en poids.

L'isostéarate de polyglycéryle-4 comprend 4 motifs d'oxyde d'éthylène. Il peut être présent en une teneur telle que le rapport pondéral de l'alkyl C₈-C₂₂ diméthicone copolyol par rapport à l'isostéarate de polyglycéryle-4 soit supérieur ou égal à 2, de préférence supérieur ou égal à 3.

Les pigments enrobés hydrophobes présents dans l'émulsion selon l'invention sont des pigments traités en surface avec un agent hydrophobe pour les rendre compatibles avec la phase grasse de l'émulsion, notamment pour qu'ils aient une bonne mouillabilité avec les huiles de la phase grasse. Ainsi, ces pigments traités sont bien dispersés dans la phase grasse.

Les pigments destinés à être enrobés peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de dioxyde de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges. On utilise de préférence des pigments d'oxydes de fer ou de dioxyde de titane.

L'agent de traitement hydrophobe peut ëtre choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le triisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine

Le terme alkyl mentionné dans les composés cités précédement désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Les pigments enrobés hydrophobes peuvent être présents en une teneur allant de 5 % à 20 % en poids, par rapport au poids total de la composition, de préférence en une teneur au moins égale à 8 % en poids, notamment allant de 8 % à 15 % en poids.

La phase grasse de l'émulsion selon l'invention comprend avantageusement au moins une huile.

L'émulsion comprend avantageusement de 20 % à 42 % en poids d'huile, par rapport au poids total de la compositon, et de préférence de 30 % à 38 % en poids.

L'émulsion selon l'invention comprend de préférence au moins une huile de silicone, en particulier une huile de silicone volatile.

L'huile peut être choisie parmi les huiles d'origine minérale, animale, végétale ou synthétique, carbonées, hydrocarbonées et/ou siliconées, et leurs mélanges.

On peut citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, d'arara, de colza, de tournesol, de coton, d'abricot, de ricin, d'avocat, de jojoba, d'olive
ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique
ou linolénique, l'alcool isostéarique ou l'octyl dodécanol.

On peut citer également les huiles siliconées telles que les polydiméthylsiloxane (PDMS), éventuellement phénylées telles que les phényltriméthicones, ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, et leurs mélanges.

Avantageusement, on peut utiliser au moins une huile volatile à température ambiante. Par huile volatile, on entend une huile susceptible de s'évaporer de la peau, à température ambiante en moins d'une heure. De préférence, l'huile volatile a une viscosité allant de 0,5 à 25 centistokes à 25°C.

Ces huiles peuvent être des huiles hydrocarbonées, des huiles siliconées comportant éventuellement des groupements alkyle ou alkoxy en bout de chaîne siliconée
ou pendante. L'huile volatile est de préférence une huile volatile siliconée.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. On peut ainsi citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane et/ou leurs mélanges.

Comme huile hydrocarbonée volatile, on peut citer les isoparaffines en C₈-C₁₆ telles que l'isooctane, l'isododécane, l'isodécane, l'heptane, l'isohexadécane et/ou leurs mélanges.

Ces huiles volatiles peuvent être présentes dans la composition selon l'invention à une teneur allant de 20 % à 45 % en poids, de préférence de 25 à 40 %, et encore préférentiellement de 30 à 40 %, par rapport au poids total de la composition.

La phase grasse peut également comprendre au moins un corps gras choisi parmi les cires, les gommes et/ou les corps gras pâteux, qui peuvent être d'origine végétale, animale, minérale ou de synthèse, voire siliconé, et leurs mélanges.

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Micro-emulsions Theory and Practice", L. M. Prince Ed., Academic Press (1977), pages 21-32. Comme cire liquide à température ambiante, on peut citer l'huile de Jojoba.

Les cires peuvent être présentes à raison de 0,1 % à 10 %, en poids, par rapport au poids total de la composition.

On peut définir les composés gras pâteux à l'aide d'au moins une des propriétés physico-chimiques suivantes :
- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.

Les compositions de l'invention peuvent également comprendre au moins une alkyl, alcoxy ou phényl-diméthicone telle que, par exemple le produit vendu sous la dénomination de "Abil wax 2440" par la Société GOLDSCHMIDT.

Les compositions selon l'invention peuvent également comprendre au moins une résine de silicone comprenant une combinaison des unités R₃SiO_{1/2} , R₂SiO_{2/2} , RSiO_{3/2} et SiO_{4/2}, dans lesquelles R désigne un radical alkyle ayant de 1 à 6 atomes de carbone.

L'émulsion selon l'invention peut comprendre, en outre, un agent épaississant de la phase grasse. L'agent épaississant peut être choisi parmi :
- les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX.
- la silice pyrogénée hydrophobe, qui est une silice pyrogénée modifiée chimiquement en surface par réaction chimique générant une diminution du nombre de groupes silanol. On peut notamment substituer des groupes silanol par des groupements hydrophobes.
   Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812® " par la société Degussa, "CAB-O-SIL TS-530® " par la société Cabot.
- des groupements diméthylsilyloxyl ou polydiméthylsiloxane, qui sont notamment obtenus par traitement de silice pyrogénée en présence de polydiméthylsiloxane
ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972® ", "AEROSIL R974® " par la société Degussa, "CAB-O-SIL TS-610® ", "CAB-O-SIL TS-720® " par la société Cabot.

La silice pyrogénée présente de préférence une taille de particules pouvant être nanométrique à micrométrique, par exemple allant d'environ de 5 à 200 nm.

L'agent épaississant de la phase grasse peut être présent en une teneur allant de 0,1% à 5% en poids, par rapport au poids total de la composition, et mieux de 0,4% à 3% en poids.

La phase grasse, incluant l'alkyl C₈-C₂₂ diméthicone copolyol et l'isostéarate de polyglycéryle-4 peut représenter de 22 % à 50 % en poids, par rapport au poids total de la composition, de préférence de 25 % à 45 % en poids, et préférentiellement de 30 % à 40 % en poids.

La phase aqueuse comprend de l'eau. L'eau peut être une eau florale telle que l'eau de bleuet et/ou une eau minérale telle que l'eau de VITTEL, l'eau de LUCAS
ou l'eau de LA ROCHE POSAY et/ou une eau thermale.

La phase aqueuse peut également comprendre des solvants autres que l'eau comme par exemple les alcools primaires tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges.

La phase aqueuse peut comprendre en outre des agents de stabilisation , par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

De préférence, la phase aqueuse est présente dans l'émulsion selon l'invention en une teneur allant 30 % à 75 %, en poids, de préférence allant de 35 % à 50 % en poids, par rapport au poids total de la composition.

L'émulsion selon l'invention peut comprendre des charges. Par charges, il faut comprendre des particules incolores ou blanches, minérales ou de synthèse, lamellaires ou non lamellaires.

Les charges peuvent être présentes dans l'émulsion en une teneur allant de 0,1 % à 15 % en poids, par rapport au poids total de l'émulsion, de préférence 0,1 % à 10 %. On peut citer notamment le talc, le mica, la silice, le kaolin, l'amidon, le nitrure de bore, le carbonate de calcium, le carbonate ou l'hydrocarbonate de magnésium, la cellulose microcristalline, les poudres de polymères synthétiques tels que le polyéthylène, les polyesters, les polyamides tels que ceux vendus sous la dénomination commerciale de "Nylon", le polytétrafluoroéthylène ("Téflon") et les poudres de silicone.

Avantageusement, l'émulsion selon l'invention est fluide (s'écoule sous son propre poids à la température ambiante) et peut avoir une viscosité, mesurée à 25°C, à une vitesse de cisaillement de 200 min⁻¹ (200 tours par minute, soit une fréquence de 50 Hz), allant de 0,5 à 3,2 Pa.s (5 à 32 poises), et de préférence allant de 0,6 à 1,5 Pa.s (6 à 15 poises). Une telle viscosité permet une application facile de l'émulsion, et l'obtention d'un maquillage homogène, uniforme et sans traces. La viscosité est mesurée à 25 °C avec un viscosimètre CONTRAVES type TV équipé d'un mobile n°3, la mesure étant effectuée après 10 minutes de rotation du mobile (temps au bout duquel on observe une stabilisation de la viscosité et de la vitesse de rotation du mobile), à un cisaillement de 200 min⁻¹ (200 tours par minute).

De façon connue, toutes les compositions de l'invention peuvent contenir un ou plusieurs des adjuvants habituels dans les domaines cosmétique et dermatologique, des agents gélifiants et/ou épaississants hydrophiles ou lipophiles ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des agents filmogènes ; des colorants solubles, et leurs mélanges. Les quantités de ces différents adjuvants sont celles classiquement utilisées dans les domaines considérés.

Comme actifs utilisables dans la composition de l'invention, on peut citer par exemple les agents hydratants tels que les hydrolysats de protéines et les polyols comme la glycérine, les glycols comme les polyéthylène glycols, et les dérivés de sucre ; les extraits naturels ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; l'acide salicylique et ses dérivés ; les alphahydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les enzymes ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents tenseurs ; et leurs mélanges.

Les filtres solaires (ou filtres U.V.) peuvent être choisis parmi les filtres organiques, les filtres physiques et leurs mélanges.

Comme filtres solaires chimiques utilisables dans la composition de l'invention, la composition de l'invention peut comprendre tous les filtres UVA et UVB utilisables dans le domaine cosmétique.
Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX ;
(3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
(4) les dérivés de l'acide p-aminobenzoïque ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple :
(1) les dérivés de dibenzoylméthane, en particulier le 4-(ter.-butyl) 4'-méthoxy dibenzoylméthane commercialisé par la société Givaudan sous la dénomination PARSOL 1789 ;
(2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
(3) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL M40 par la société BASF ;
   - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer ;
(7) les dérivés siliciés de benzimidazolyl-benzazoles N-substitués ou de benzofuranyl-benzazoles, et en particulier :
   - le 2-[1-[3-[1,3,3,3-tétraméthyl-1-[(triméthylsilyl)oxy]disiloxanyl]propyl]-1H-benzimidazol-2-yl]-benzoxazole ;
   - le 2-[1-[3-[1,3,3,3-tétraméthyl-1-[(triméthylsilyl)oxy]disiloxanyl]propyl]-1H-benzimidazol-2-yl]-benzothiazole ;
   - le 2-[1-(3-trimethylsilanyl-propyl)-1H-benzimidazol-2-yl]-benzoxazole ;
   - le 6-méthoxy-1,1'-bis-(3-triméthylsilanyl-propyl)-1H,1'H-[2,2']bibenzimidazolyl-benzoxazole ;
   - le 2-[1-(3-trimethylsilanyl-propyl)-1H-benzimidazol-2-yl]-benzothiazole ; qui sont décrits dans la demande de brevet EP-A-1 028 120 ;
(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) leurs mélanges.

On peut aussi utiliser un mélange de plusieurs de ces filtres et un mélange de filtres UVB et de filtres UVA et aussi des mélanges avec des filtres physiques.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm.
Ces pigments sont de préférence traités de manière à rendre leur surface hydrophobe; ce traitement peut être effectué selon les méthodes connues de l'homme du métier ; les pigments peuvent notamment être enrobés par des composés siliconés tels que des PDMS et/ou par des polymères.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

L'invention est illustrée plus de détail dans les exemples suivants.

### Exemple 1 :

On a préparé un fond de teint sous forme d'émulsion eau-dans-huile ayant la composition suivante :

| Phase huileuse : | |
|---|---|
| Palmitate d'isostéaryle | 6 g |
| Cyclopentasiloxane | 30 g |
| Cetyl diméthicone copolyol (Abil® EM 90 de la société GOLDSCHMIDT) | 2,7 g |
| Isostéarate de polyglycéryle-4 | 0,9 g |
| Oxydes de fer enrobés de sel disodique de stéaroyl glutamate | 2 g |
| Oxyde de titane enrobé de sel disodique de stéaroyl glutamate | 9 g |
| Poudre de nylon | 5 g |
| Hectorite | 0,5 g |
| Silice pyrogénée hydrophobe (Aérosil R 972 ) | 0,35 g |
| Microsphères creuses (Expancel) | 0,35 g |

| Phase aqueuse : | | |
|---|---|---|
| Butylène glycol | | 5 g |
| Sulfate de magnésium | | 1 g |
| PEG 20 | | 1,7 g |
| Conservateurs | qs | |
| Eau | qsp | 100 g |

L'émulsion est préparée à température ambiante d'une part en mélangeant les pigments dans une partie du cyclopentasiloxane, d'autres part en mélangeant les autres huiles avec les tensioactifs et en y dispersant l'hectorite, puis on ajoute le mélange de pigments et des charges aux autres constituants de la phase grasse mélangés. On prépare ensuite le mélange des constituants de la phase aqueuse que l'on verse dans le mélange de la phase grasse, sous agitation selon les moyens connus pour obtenir au final l'émulsion.

Ce fond de teint est stable après stockage à température ambiante (25°C) pendant 4 mois, voire même jusqu'à 8 mois. Il s'applique facilement sur la peau avec une bonne sensation au toucher, sèche rapidement après l'application, et le maquillage obtenu présente une bonne homogénéité de la couleur.

## Revendications

1. Fond de teint sous forme d'émulsion eau-dans-huile comprenant une phase huileuse, une phase aqueuse, un alkyl C₈-C₂₂ diméthiconecopolyol, au moins 5 % en poids, par rapport au poids total de la composition, de pigments enrobés hydrophobes, la composition contenant de l'isostéarate de polyglycéryle-4 en une quantité telle que le rapport pondéral de l'alkyl C₈-C₂₂ diméthiconecopolyol par rapport à l'isostéarate de polyglycéryle-4 est supérieur ou égal à 2.

2. Fond de teint selon la revendication 1, **caractérisé par le fait que** l'alkyl C₈-C₂₂ diméthicone copolyol est un composé de formule (I) suivante : 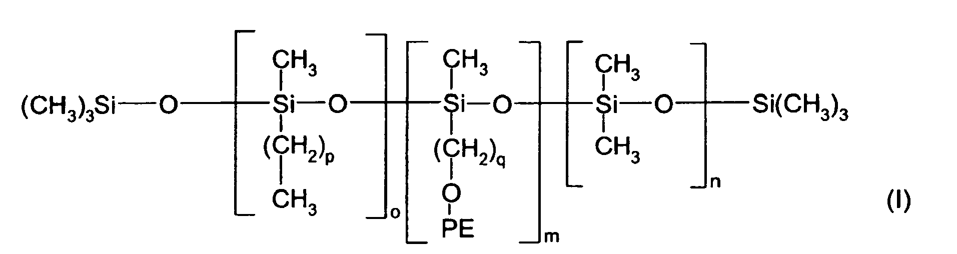 dans laquelle :
- PE représente (-C₂H₄O)ₓ-(C₃H₆O)_{y}-R, R étant choisi parmi un atome d'hydrogène et un radical alkyle de 1 à 4 atomes de carbone, x allant de 0 à 100 et y allant de 0 à 80, x et y n'étant pas simultanémént 0
- m va de 1 à 40
- n va de 10 à 200
- o va de 1 à 100
- pvade7à21
- q va de 0 à 4

3. Fond de teint selon la revendication 2, **caractérisé par le fait que** R = H ; m = 1 à 10 ; n = 10 à 100 ; o = 1 à 30 ; p = 15 ; q = 3

4. Fond de teint selon l'une quelconque des revendications précédentes, **caractérisé par le fait que** l'alkyl C₈-C₂₂ diméthicone copolyol est le cétyl diméthicone copolyol.

5. Fond de teint selon l'une quelconque des revendications précédentes, **caractérisé par le fait que** l'alkyl C₈-C₂₂ diméthicone copolyol est présent en une teneur allant de 2 % à 10 % en poids, par rapport au poids total de l'émulsion, et de préférence allant de 2,5 % à 5 % en poids.

6. Fond de teint selon l'une quelconque des revendications précédentes, **caractérisé par le fait que** l'isostéarate de polyglycéryle-4 est présent en une teneur telle que le rapport pondéral de falkyl C₈-C₂₂ diméthicone copolyol par rapport à l'isostéarate de polyglycéryle-4 soit supérieur ou égal à 3.

7. Fond de teint selon l'une quelconque des revendications précédentes, **caractérisé par le fait que** les pigments enrobés hydrophobes sont choisis parmi les oxydes métalliques, le violet de manganèse, l'ultramarine bleue, le bleu de prusse, le bleu outremer, le bleu ferrique, l'oxychlorure de bismuth, la nacre, le mica recouvert de dioxyde de titane ou d'oxychlorure de bismuth, les pigments nacrés colorés, et leurs mélanges.

8. Fond de teint selon l'une quelconque des revendications précédentes, **caractérisé par le fait que** les pigments enrobés hydrophobes sont choisis parmi les oxydes de fer et les dioxydes de titane.

9. Fond de teint selon l'une quelconque des revendications précédentes, **caractérisé par le fait que** les pigments enrobés hydrophobes sont traités avec un agent hydrophobe choisi parmi les silicones, les acides gras, les savons métalliques, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le triisostéaryle titanate d'isopropyle, et leurs mélanges.

10. Fond de teint selon la revendication 9, **caractérisé par le fait que** les acides aminés N-acylés comprennent un groupe acyle ayant de 8 à 22 atomes de carbones.

11. Fond de teint selon l'une quelconque des revendications précédentes, **caractérisé par le fait que** les pigments enrobés hydrophobes sont présents en une teneur allant de 5 % à 20 % en poids, par rapport au poids total de l'émulsion, de préférence en une teneur au moins égale à 8 % en poids, et préférentiellement allant de 8 % à 15 % en poids.

12. Fond de teint selon l'une quelconque des revendications précédentes, **caractérisé par le fait qu'**il comprend une huile de silicone.

13. Fond de teint selon la revendication 12, **caractérisé par le fait que** l'huile de silicone est choisie parmi les polydiméthylsiloxanes, les huiles de silicones phénylées, les polysiloxanes modifiés par des acides gras, des alcools gras ou des polyoxyalkylènes, et leurs mélanges.

14. Fond de teint selon l'une quelconque des revendications précédentes, **caractérisé par le fait qu'**il comprend une huile de silicone volatile.

15. Fond de teint selon la revendication 14, **caractérisé par le fait que** l'huile de silicone volatile est choisie parmi l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane, et leurs mélanges.

16. Fond de teint selon l'une quelconque des revendications précédentes, **caractérisé par le fait qu'**il comprend une huile hydrocarbonée.

17. Fond de teint selon la revendication 16, **caractérisé par le fait que** l'huile hydrocarbonée est choisie parmi l'huile de paraffine ou de vaseline, l'huile de vison, de tortue, de soja, le perhydrosqualène, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; les esters d'acide lanolique, d'acide oléique, d'acide laurique, d'acide stéarique ; le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; l'acide myristique, l'acide palmitique, l'acide stéarique, l'acide béhénique, l'acide oléique, l'acide linoléique, l'acide linolénique, l'acide isostéarique ; le cétanol, l'alcool stéarylique ou l'alcool oléique, l'alcool linoléique ou linolénique, l'alcool isostéarique, l'octyl dodécanol.

18. Fond de teint selon l'une quelconque des revendications précédentes, **caractérisé par le fait qu'**il comprend une huile hydrocarbonée volatile.

19. Fond de teint selon la revendication 18, **caractérisé par le fait que** l'huile hydrocarbonée volatile est une isoparaffine en C₈-C₁₆, choisie parmi l'isooctane, l'isododécane, l'isodécane, l'heptane, l'isohexadécane, et leurs mélanges.

20. Fond de teint selon l'une quelconque des revendications précédentes, **caractérisé par le fait qu'**il comprend de 20 % à 42 % en poids d'huile, par rapport au poids total de la composition, et de préférence de 30 % à 38 % en poids.

21. Fond de teint selon l'une quelconque des revendications 14, 15, 18, 19, **caractérisé par le fait qu'**il comprend de 20 % à 45 % en poids d'huile volatile, par rapport au poids total de la composition, de préférence de 25 % à 40 % en poids, et préférentiellement de 30 % à 40 % en poids.

22. Fond de teint selon l'une quelconque des revendications précédentes, **caractérisé par le fait qu'**il comprend un corps gras choisi parmi les cires, les gommes, les corps gras pâteux, et leurs mélanges.

23. Fond de teint selon l'une quelconque des revendications précédentes, **caractérisé par le fait qu'**il comprend un agent épaississant de la phase grasse.

24. Fond de teint selon la revendication 23, **caractérisé par le fait que** l'agent épaississant est choisi parmi les argiles organomodifiées et la silice pyrogénée hydrophobe.

25. Fond de teint selon la revendication 23 ou 24, **caractérisé par le fait que** l'agent épaississant de la phase grasse est présent en une teneur allant de 0,1% à 5% en poids, par rapport au poids total de la composition, et mieux de 0,4% à 3% en poids.

26. Fond de teint selon l'une quelconque des revendications précédentes, **caractérisé par le fait que** la phase huileuse représente de 22 % à 50 % en poids, par rapport au poids total de la composition, de préférence de 25 % à 45 % en poids, et préférentiellement de 30 % à 40 % en poids.

27. Fond de teint selon l'une quelconque des revendications précédentes, **caractérisé par le fait que** la phase aqueuse est présente en une teneur allant 30 % à 75 %, en poids, de préférence allant de 35 % à 50 % en poids, par rapport au poids total de la composition.

28. Fond de teint selon l'une quelconque des revendications précédentes, **caractérisé par le fait que** la phase aqueuse comprend un solvant choisi parmi les alcools primaires, les glycols, les éthers de glycol, et leurs mélanges et/ou un agent de stabilisation.

29. Fond de teint selon l'une quelconque des revendications précédentes, **caractérisé par le fait qu'**il comprend des charges.

30. Fond de teint selon la revendication 29, **caractérisé par le fait que** les charges sont choisies parmi le talc, le mica, la silice, le kaolin, l'amidon, le nitrure de bore, le carbonate de calcium, le carbonate ou l'hydrocarbonate de magnésium, la cellulose microcristalline, les poudres de polyéthylène, les polyesters, les polyamides, le polytétrafluoroéthylène ("Téflon"), les poudres de silicone, et leurs mélanges.

31. Fond de teint selon la revendication 29 ou 30, **caractérisé par le fait que** les charges sont présentes en une teneur allant de 0,1 % à 15 % en poids, par rapport au poids total de l'émulsion, de préférence 0,1 % à 10 %.

32. Fond de teint selon l'une quelconque des revendications précédentes, **caractérisé par le fait qu'**il comprend en outre au moins un additif choisi parmi les agents gélifiants, les épaississants hydrophiles ou lipophiles, les agents hydratants ; les émollients ; les actifs hydrophiles ou lipophiles ; les agents anti-radicaux libres ; les séquestrants ; les antioxydants ; les conservateurs ; les agents alcanisants ou acidifiants ; les parfums ; les agents filmogènes ; les colorants solubles , et leurs mélanges.

33. Fond de teint selon l'une quelconque des revendications précédentes, **caractérisée par le fait que** la composition a une viscosité, mesurée à 25°C, à une vitesse de cisaillement de 200 tours par minute, allant de 0,5 à 3,2 Pa.s, de préférence allant de 0,6 à 1,5 Pa.

34. Procédé cosmétique de maquillage non thérapeutique de la peau comprenant l'application sur la peau d'un fond de teint selon l'une quelconque des revendications précédentes.

35. Utilisation d'un alkyl C8-C22 diméthiconecopolyol dans une composition de fond de teint sous forme d'émulsion eau-dans-huile contenant au moins 5 % en poids, par rapport au poids total de la composition, de pigments enrobés hydrophobes, la composition contenant de l'isostéarate de polyglycéryle-4 en une quantité telle que le rapport pondéral de l'alkyl C8-C22 diméthiconecopolyol par rapport à l'isostéarate de polyglycéryle-4 est supérieur ou égal à 2, pour obtenir une émulsion stable et/ou homogène et/ou s'appliquant facilement sur la peau, et/ou pour obtenir un maquillage homogène de la peau.

## Claims

1. Foundation in the form of a water-in-oil emulsion comprising an oily phase, an aqueous phase, a C₈₋C₂₂ alkyl dimethicone copolyol, at least 5% by weight, relative to the total weight of the composition, of hydrophobic coated pigments, the composition containing polyglyceryl-4 isostearate in a quantity such that the weight ratio of the C₈₋C₂₂ alkyl dimethicone copolyol to the polyglyceryl-4 isostearate is greater than or equal to 2.

2. Foundation according to Claim 1, **characterized in that** the C₈-C₂₂ alkyl dimethicone copolyol is a compound of the following formula (I): 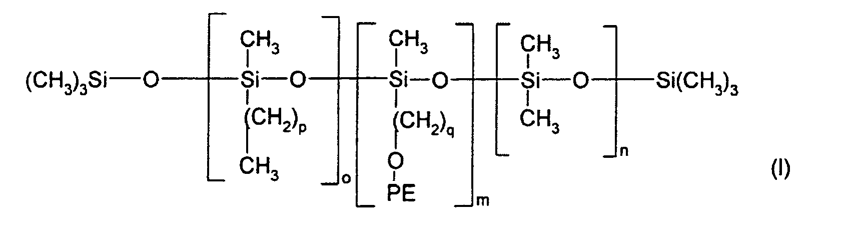 in which:
- PE represents (-C₂H₉O)ₓ-(C₃H₆O)_{y}-R, R being chosen from a hydrogen atom and an alkyl radical of 1 to 4 carbon atoms, x ranging from 0 to 100 and y ranging from 0 to 80, x and y not being simultaneously 0
- m ranges from 1 to 40
- n ranges from 10 to 200
- o ranges from 1 to 100
- p ranges from 7 to 21
- q ranges from 0 to 4.

3. Foundation according to Claim 2, **characterized in that** R = H; m = 1 to 10; n = 10 to 100; o = 1 to 30; p = 15; q = 3.

4. Foundation according to any one of the preceding claims, **characterized in that** the C₈-C₂₂ alkyl dimethicone copolyol is cetyl dimethicone copolyol.

5. Foundation according to any one of the preceding claims, **characterized in that** the C₈-C₂₂ alkyl dimethicone copolyol is present in an amount ranging from 2% to 10% by weight, relative to the total weight of the emulsion, and preferably ranging from 2.5% to 5% by weight.

6. Foundation according to any one of the preceding claims, **characterized in that** the polyglyceryl-4 isostearate is present in an amount such that the weight ratio of the C₈-C₂₂ alkyl dimethicone copolyol to the polyglyceryl-4 isostearate is greater than or equal to 3.

7. Foundation according to any one of the preceding claims, **characterized in that** the hydrophobic coated pigments are chosen from metal oxides, manganese violet, ultramarine blue, Prussian blue, ferric blue, bismuth oxychloride, pearl, mica coated with titanium dioxide or with bismuth oxychloride, coloured pearlescent pigments, and mixtures thereof.

8. Foundation according to any one of the preceding claims, **characterized in that** the hydrophobic coated pigments are chosen from iron oxides and titanium dioxides.

9. Foundation according to any one of the preceding claims, **characterized in that** the hydrophobic coated pigments are treated with a hydrophobic agent chosen from silicones, fatty acids, metal soaps, perfluoroalkyl phosphates, perfluoroalkylsilanes, perfluoroalkylsilazanes, polyhexafluoropropylene oxides, polyorganosiloxanes comprising perfluoroalkyl perfluoropolyether groups, amino acids; N-acylated amino acids or their salts; lecithin, isopropyl triisostearyl titanate, and mixtures thereof.

10. Foundation according to Claim 9, **characterized in that** the N-acylated amino acids comprise an acyl group having from 8 to 22 carbon atoms.

11. Foundation according to any one of the preceding claims, **characterized in that** the hydrophobic coated pigments are present in an amount ranging from 5% to 20% by weight, relative to the total weight of the emulsion, preferably in an amount at least equal to 8% by weight, and preferably ranging from 8% to 15% by weight.

12. Foundation according to any one of the preceding claims, **characterized in that** it comprises a silicone oil.

13. Foundation according to Claim 12, **characterized in that** the silicone oil is chosen from polydimethylsiloxanes, phenylated silicone oils, polysiloxanes modified with fatty acids, fatty alcohols or polyoxyalkylenes, and mixtures thereof.

14. Foundation according to any one of the preceding claims, **characterized in that** it comprises a volatile silicone oil.

15. Foundation according to Claim 14, **characterized in that** the volatile silicone oil is chosen from octamethylcyclotetrasiloxane, decamethylcyclopentasiloxane, hexadecamethylcyclohexasiloxane, heptamethylhexyltrisiloxane, heptamethyloctyltrisiloxane, and mixtures thereof.

16. Foundation according to any one of the preceding claims, **characterized in that** it comprises a hydrocarbonaceous oil.

17. Foundation according to Claim 16, **characterized in that** the hydrocarbonaceous oil is chosen from paraffin oil or liquid paraffin, vison oil, turtle oil, soya bean oil, perhydrosqualene, sweet almond oil, calophyllum oil, palm oil, grapeseed oil, sesame oil, maize oil, arara oil, rapeseed oil, sunflower oil, cottonseed oil, apricot oil, castor oil, avocado oil, jojoba oil, olive oil or cereal germ oil; esters of lanolic acid, oleic acid, lauric acid and stearic acid; isopropyl myristate, isopropyl palmitate, butyl stearate, hexyl laurate, diisopropyl adipate, isononyl isononanoate, 2-ethylhexyl palmitate, 2-hexyldecyl laurate, 2-octyldecyl palmitate, 2-octyldodecyl myristate or lactate, 2-diethylhexyl succinate, diisostearyl malate, glycerine or diglycerine triisostearate; myristic acid, palmitic acid, stearic acid, behenic acid, oleic acid, linoleic acid, linolenic acid or isostearic acid; cetanol, stearyl alcohol or oleyl alcohol, linoleyl or linolenyl alcohol, isostearyl alcohol or octyldodecanol.

18. Foundation according to any one of the preceding claims, **characterized in that** it comprises a volatile hydrocarbonaceous oil.

19. Foundation according to Claim 18, **characterized in that** the volatile hydrocarbonaceous oil is a C₈-C₁₆ isoparaffin chosen from isooctane, isododecane, isodecane, heptane, isohexadecane, and mixtures thereof.

20. Foundation according to any one of the preceding claims, **characterized in that** it comprises from 20% to 42% by weight of oil, relative to the total weight of the composition, and preferably from 30% to 38% by weight.

21. Foundation according to any one of Claims 14, 15, 18 and 19, **characterized in that** it comprises from 20% to 45% by weight of volatile oil, relative to the total weight of the composition, preferably from 25% to 40% by weight, and preferably from 30% to 40% by weight.

22. Foundation according to any one of the preceding claims, **characterized in that** it comprises a fatty substance chosen from waxes, gums, pasty fatty substances, and mixtures thereof.

23. Foundation according to any one of the preceding claims, **characterized in that** it comprises a fatty phase thickening agent.

24. Foundation according to Claim 23, **characterized in that** the thickening agent is chosen from organomodified clays and hydrophobic pyrogenic silica.

25. Foundation according to Claim 23 or 24,
**characterized in that** the fatty phase thickening agent is present in an amount ranging from 0.1% to 5% by weight, relative to the total weight of the composition, and even better from 0.4% to 3% by weight.

26. Foundation according to any one of the preceding claims, **characterized in that** the oily phase represents from 22% to 50% by weight, relative to the total weight of the composition, preferably from 25% to 45% by weight, and preferably from 30% to 40% by weight.

27. Foundation according to any one of the preceding claims, **characterized in that** the aqueous phase is present in an amount ranging from 30% to 75% by weight, preferably ranging from 35% to 50% by weight, relative to the total weight of the composition.

28. Foundation according to any one of the preceding claims, **characterized in that** the aqueous phase comprises a solvent chosen from primary alcohols, glycols, glycol ethers, and mixtures thereof, and/or a stabilizing agent.

29. Foundation according to any one of the preceding claims, **characterized in that** it comprises fillers.

30. Foundation according to Claim 29, **characterized in that** the fillers are chosen from talc, mica, silica, kaolin, starch, boron nitride, calcium carbonate, magnesium carbonate or hydrocarbonate, microcrystalline cellulose, polyethylene powders, polyesters, polyamides, polytetrafluoroethylene ("Teflon"), silicone powders, and mixtures thereof.

31. Foundation according to Claim 29 or 30,
**characterized in that** the fillers are present in an amount ranging from 0.1% to 15% by weight, relative to the total weight of the emulsion, preferably 0.1% to 10%.

32. Foundation according to any one of the preceding claims, **characterized in that** it comprises in addition at least one additive chosen from gelling agents, hydrophilic or lipophilic thickening agents, moisturizing agents; emollients; hydrophilic or lipophilic active agents; anti-free radical agents; sequestrants; antioxidants; preservatives; basifying or acidifying agents; perfumes; film-forming agents; soluble colorants, and mixtures thereof.

33. Foundation according to any one of the preceding claims, **characterized in that** the composition has a viscosity, measured at 25°C, at a shear rate of 200 revolutions per minute, ranging from 0.5 to 3.2 Pa.s, preferably ranging from 0.6 to 1.5 Pa.s.

34. Cosmetic method for the non-therapeutic application of make-up to the skin which comprises the application to the skin of a foundation according to any one of the preceding claims.

35. Use of a C₈-C₂₂ alkyl dimethicone copolyol in a foundation composition in the form of a water-in-oil emulsion containing at least 5% by weight, relative to the total weight of the composition, of hydrophobic coated pigments, the composition containing polyglyceryl-4 isostearate in a quantity such that the weight ratio of the C₈-C₂₂ alkyl dimethicone copolyol to the polyglyceryl-4 isostearate is greater than or equal to 2, in order to obtain an emulsion which is stable and/or which is homogeneous and/or which can be easily applied to the skin, and/or in order to obtain a homogeneous application of make-up to the skin.

## Patentansprüche

1. Make-up, das in Form einer Wasser-in-Öl-Emulsion vorliegt und eine Ölphase, eine wässrige Phase, ein C₈₋₂₂-Alkyldimethiconcopolyol und mindestens 5 Gew.-% hydrophobe umhüllte Pigmente, bezogen auf das Gesamtgewicht der Zusammensetzung, enthält, wobei die Zusammensetzung Tetraglycerylmonoisostearat in einer solchen Menge enthält, dass das Gewichtsverhältnis des C₈₋₂₂-Alkyldimethiconcopolyols und des Tetraglycerylmonoisostearats 2 beträgt oder darüber liegt.

2. Make-up nach Anspruch 1, **dadurch gekennzeichnet, dass** das C₈₋₂₂-Alkyldimethiconcopolyol eine Verbindung der folgenden Formel (I) ist: 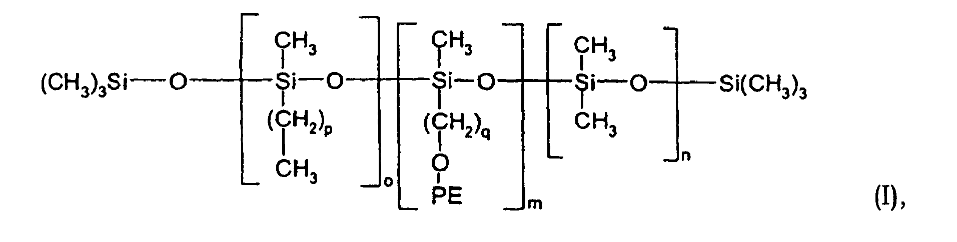 worin bedeuten:
- PE die Gruppe (-C₂H₄O)ₓ-(C₃H₆O)_{y}-R, wobei R unter einem Wasserstoffatom oder einer Alkylgruppe mit 1 bis 4 Kohlenstoffatomen ausgewählt ist, x im Bereich von 0 bis 100 und y im Bereich von 0 bis 80 liegt, wobei x und y nicht gleichzeitig 0 bedeuten,
- m liegt im Bereich von 1 bis 40,
- n liegt im Bereich von 10 bis 200,
- o liegt im Bereich von 1 bis 100,
- p liegt im Bereich von 7 bis 21,
- q liegt im Bereich von 0 bis 4.

3. Make-up nach Anspruch 2, **dadurch gekennzeichnet, dass** R = H; m = 1 bis 10; n = 10 bis 100; o = 1 bis 30; p = 15; q = 3.

4. Make-up nach einem der vorhergehenden Ansprüche, **dadurch gekennzeichnet, dass** das C₈₋₂₂-Alkyldimethiconcopolyol das Cetyldimethiconcopolyol ist.

5. Make-up nach einem der vorhergehenden Ansprüche, **dadurch gekennzeichnet, dass** das C₈₋₂₂-Alkyldimethiconcopolyol in einer Menge von 2 bis 10 Gew.-%, bezogen auf das Gesamtgewicht der Emulsion, und vorzugsweise 2,5 bis 5 Gew.-% enthalten ist.

6. Make-up nach einem der vorhergehenden Ansprüche, **dadurch gekennzeichnet, dass** das Tetraglycerylmonoisostearat in einer Menge vorliegt, die so ist, dass das Gewichtsverhältnis von C₈₋₂₂-Alkyldimethiconcopolyol und Tetraglycerylmonoisostearat mindestens 3 beträgt.

7. Make-up nach einem der vorhergehenden Ansprüche, **dadurch gekennzeichnet, dass** die hydrophoben umhüllten Pigmente unter Metalloxiden, Manganviolett, Ultramarinblau, Berliner Blau, Ultramarinpigmenten, Eisenblau, Bismutoxidchlorid, Perlmutt, mit Titandioxid oder Bismutoxidchlorid überzogenen Glimmerpigmenten, farbigen Perlglanzpigmenten und deren Gemischen ausgewählt sind.

8. Make-up nach einem der vorhergehenden Ansprüche, **dadurch gekennzeichnet, dass** die hydrophoben umhüllten Pigmente unter den Oxiden von Eisen und den Oxiden von Titan ausgewählt sind.

9. Make-up nach einem der vorhergehenden Ansprüche, **dadurch gekennzeichnet, dass** die umhüllten hydrophoben Pigmente mit einem hydrophoben Stoff behandelt sind, der unter den Siliconen, Fettsäuren, Metallseifen, Perfluoralkylphosphaten, Perfluoralkylsilanen, Perfluoralkylsilazanen, Hexafluorpropylenpolyoxiden, Polyorganosiloxanen mit Perfluoralkylperfluorpolyethergruppen, Aminosäuren; N-acylierten Aminosäuren oder deren Salzen; Lecithin, Isopropyltrüsostearyltitanat und deren Gemischen ausgewählt sind.

10. Make-up nach Anspruch 9, **dadurch gekennzeichnet, dass** die N-acylierten Aminosäuren eine Acylgruppe mit 8 bis 22 Kohlenstoffatomen enthalten.

11. Make-up nach einem der vorhergehenden Ansprüche, **dadurch gekennzeichnet, dass** die hydrophoben umhüllten Pigmente in einer Menge von 5 bis 20 Gew.-%, bezogen auf das Gesamtgewicht der Emulsion, vorzugsweise in einer Menge von mindestens 8 Gew.-% und noch bevorzugter von 8 bis 15 Gew.-% enthalten sind.

12. Make-up nach einem der vorhergehenden Ansprüche, **dadurch gekennzeichnet, dass** es ein Siliconöl enthält.

13. Make-up nach Anspruch 12, **dadurch gekennzeichnet, dass** das Siliconöl unter den Polydimethylsiloxanen, phenylierten Siliconölen, mit Fettsäuren, Fettalkoholen oder Polyalkylenoxiden modifizierten Polysiloxanen und deren Gemischen ausgewählt ist.

14. Make-up nach einem der vorhergehenden Ansprüche, **dadurch gekennzeichnet, dass** es ein flüchtiges Silicon enthält.

15. Make-up nach Anspruch 14, **dadurch gekennzeichnet, dass** das flüchtige Siliconöl unter Octamethylcyclotetrasiloxan, Decamethylcyclopentasiloxan, Hexadecamethylcyclohexasiloxan, Heptamethylhexyltrisiloxan, Heptamethyloctyltrisiloxan und deren Gemischen ausgewählt ist.

16. Make-up nach einem der vorhergehenden Ansprüche, **dadurch gekennzeichnet, dass** es ein Kohlenwasserstofföl enthält.

17. Make-up nach Anspruch 16, **dadurch gekennzeichnet, dass** das Kohlenwasserstofföl ausgewählt ist unter Paraffinöl oder Vaseline, Nerzöl, Schildkrötenöl, Sojaöl, Perhydrosqualen, Süßmandelöl, Calophyllumöl, Palmöl, Traubenkernöl, Sesamöl, Maisöl, Araraöl, Rapsöl, Sonnenblumenöl, Baumwollsamenöl, Aprikosenkernöl, Ricinusöl, Avocadoöl, Jojobaöl, Olivenöl oder Getreidekeimöl; Estern von Lanolinsäure, Ölsäure, Laurinsäure, Stearinsäure; Isopropylmyristat, Isopropylpalmitat, Butylstearat, Hexyllaurat, Düsopropyladipat, Isononylisononanoat, 2-Ethylhexylpalmitat, 2-Hexyldecyllaurat, 2-Octyldecylpalmitat, 2-Octyldodecylmyristat, 2-Octyldodecyllactat, 2-Diethylhexylsuccinat, Diisostearylmalat, Glyceryltriisostearat oder Diglyceryltriisostearat; Myristinsäure, Palmitinsäure, Stearinsäure, Behensäure, Ölsäure, Linolsäure, Linolensäure, Isostearinsäure; Cetanol, Stearylalkohol oder Oleylalkohol, Linolylalkohol oder Linolenylalkohol, I-sostearylalkohol, Octyldodecanol.

18. Make-up nach einem der vorhergehenden Ansprüche, **dadurch gekennzeichnet, dass** es ein flüchtiges Kohlenwasserstofföl enthält.

19. Make-up nach Anspruch 18, **dadurch gekennzeichnet, dass** das flüchtige Kohlenwasserstofföl ein C₈₋₁₆-Isoparaffin ist, das unter Isooctan, Isododecan, Isodecan, Heptan, Isohexadecan und deren Gemischen ausgewählt ist.

20. Make-up nach einem der vorhergehenden Ansprüche, **dadurch gekennzeichnet, dass** es 20 bis 42 Gew.-% Öl, bezogen auf das Gesamtgewicht der Zusammensetzung, und vorzugsweise 30 bis 38 Gew.-% Öl enthält.

21. Make-up nach einem der Ansprüche 14, 15, 18, 19, **dadurch gekennzeichnet, dass** es 20 bis 45 Gew.-% flüchtiges Öl, bezogen auf das Gesamtgewicht der Zusammensetzung, vorzugsweise 25 bis 40 Gew.-% und noch bevorzugter 30 bis 40 Gew.% Öl enthält.

22. Make-up nach einem der vorhergehenden Ansprüche, **dadurch gekennzeichnet, dass** es eine Fettsubstanz enthält, die unter den Wachsen, Gummis, pastösen Fettsubstanzen und deren Gemischen ausgewählt ist.

23. Make-up nach einem der vorhergehenden Ansprüche, **dadurch gekennzeichnet, dass** es ein Verdickungsmittel für die Fettphase enthält.

24. Make-up nach Anspruch 23, **dadurch gekennzeichnet, dass** das Verdickungsmittel unter den organomodifizierten Tonen und hydrophober pyrogener Kieselsäure ausgewählt ist.

25. Make-up nach Anspruch 23 oder 24, **dadurch gekennzeichnet, dass** das Verdickungsmittel für die Fettphase in einer Menge von 0,1 bis 5 Gew.-%, bezogen auf das Gesamtgewicht der Zusammensetzung, und besser 0,4 bis 3 Gew.-% ausgewählt ist.

26. Make-up nach einem der vorhergehenden Ansprüche, **dadurch gekennzeichnet, dass** die Ölphase 22 bis 50 Gew.-%, bezogen auf das Gesamtgewicht der Zusammensetzung, vorzugsweise 25 bis 45 Gew.-% und noch bevorzugter 30 bis 40 Gew.-% ausmacht.

27. Make-up nach einem der vorhergehenden Ansprüche, **dadurch gekennzeichnet, dass** die wässrige Phase in einer Menge von 30 bis 75 Gew.-%, vorzugsweise 35 bis 50 Gew.-%, bezogen auf das Gesamtgewicht der Zusammensetzung, enthalten ist.

28. Make-up nach einem der vorhergehenden Ansprüche, **dadurch gekennzeichnet, dass** die wässrige Phase ein Lösungsmittel, das unter den primären Alkoholen, Glykolen, Glykolethern und deren Gemischen ausgewählt ist, und/oder ein Stabilisierungsmittel enthält.

29. Make-up nach einem der vorhergehenden Ansprüche, **dadurch gekennzeichnet, dass** es Füllstoffe enthält.

30. Make-up nach Anspruch 29, **dadurch gekennzeichnet, dass** die Füllstoffe unter Talk, Glimmer, Kieselsäure, Kaolin, Stärke, Bornitrid, Calciumcarbonat, Magnesiumcarbonat, Magnesiumhydrogencarbonat, mikrokristalliner Cellulose, Pulvern von Polyethylen, Polyestern, Polyamiden, Polytetrafluorethylen ("Teflon"), Siliconpulvern und deren Gemischen ausgewählt sind.

31. Make-up nach Anspruch 29 oder 30, **dadurch gekennzeichnet, dass** die Füllstoffe in einer Menge von 0,1 bis 15 Gew.-%, bezogen auf das Gesamtgewicht der Emulsion, und vorzugsweise 0,1 bis 10 % vorliegen.

32. Make-up nach einem der vorhergehenden Ansprüche, **dadurch gekennzeichnet, dass** es ferner mindestens einen Zusatzstoff enthält, der unter den Gelbildnern, hdrophilen oder lipophilen Verdickungsmitteln, Hydratisierungsmittel; Emollientien; hydrophilen oder lipophilen Wirkstoffen; Radikalfängern für freie Radikale; Maskierungsmitteln; Antioxidantien; Konservierungsmitteln; Alkalisierungsmitteln oder Ansäuerungsmitteln; Parfums, Filmbildnern; löslichen Farbstoffen und deren Gemischen ausgewählt ist.

33. Make-up nach einem der vorhergehenden Ansprüche, **dadurch gekennzeichnet, dass** die Zusammensetzung eine bei 25 °C und einer Schergeschwindigkeit von 200 U/min gemessenen Viskosität von 0,5 bis 3,2 Pa·s und vorzugsweise 0,6 bis 1,5 Pa aufweist.

34. Nicht therapeutisches kosmetisches Verfahren zum Schminken der Haut, das das Aufbringen eines Make-ups nach einem der vorhergehenden Ansprüche auf die Haut umfasst.

35. Verwendung eines C₈₋₂₂-Alkyldimethiconcopolyols in einer Zusammensetzung für Make-up, die in Form einer Wasser-in-Öl-Emulsion vorliegt und mindestens 5 Gew.-% hydrophobe umhüllte Pigmente, bezogen auf das Gesamtgewicht der Zusammensetzung, enthält, wobei die Zusammensetzung Tetraglycerylmonoisostearat in einer solchen Menge enthält, dass das Gewichtsverhältnis des C₈₋₂₂-Alkyldimethiconcopolyols und des Tetraglycerylmonoisostearats mindestens 2 beträgt, um eine Emulsion zu erhalten, die stabil und/oder homogen ist und/oder sich leicht auf die Haut auftragen lässt, und/oder um auf der Haut eine homogene Schminke zu bilden.
